# EUROPEAN PATENT APPLICATION

(11) **EP 3 023 112 A1**
(43) Date of publication of application: **25.05.2016**
(21) Application number: 14194564.2
(22) Date of filing: 24.11.2014
(51) Int. Cl.: A61M 1/02, A61M 1/36, A61M 1/34

(54) **A blood fractioning device**

(71) Applicant: Hemanua Limited, Dublin 4 (IE)
(72) Inventor: Maher, Daniel, Dublin, 4 (IE); Stienstra, Stef, 9718 EN Groningen (NL); O'Donoghue, John, Waterford (IE); O'Neill, Liam, Cork (IE); Cooney, Brian, Waterford (IE)
(74) Representative: Isarpatent

(57) **Abstract**

A gravity based blood fractioning device comprising a component for receiving whole blood which is connected to an inlet chamber of a filter and wherein said filter possesses separate outlet chambers connecting to a receptacle for cellular blood components and a receptacle for blood plasma and wherein each receptacle is connected to said filter via a conduit and wherein the conduit connected to the cellular blood component receptacle is of sufficient length to allow said receptacle to be raised up sufficiently high as to reverse the flow of cellular blood components back through the filter a second time to obtain a second degree of filtration of the cellular blood components and thereby increase the volume of plasma extracted.

## Description

### Technical Background

Blood supply, and continuity of available donors, is a critical foundation stone of modern health systems. While whole blood donations can be transfused directly to a recipient patient, this is rare in advanced medical systems, where blood is generally separated into its various constituent components, and only the appropriate component transfused. However whole blood transfusion remains very common practice throughout many parts of the developing world.

There are many reasons to process blood and to separate it into multiple components. Red blood cells may be donated to anaemic patients or replace critical cell lost in accidents, surgery or childbirth. Plasma may be transfused into patients suffering from traumatic blood loss or from diseases that affect clotting factors. Separation processing ensures that a single donation of blood can help multiple patients.

Of particular interest is the use of convalescent plasma therapy in the treatment of infectious diseases. Infections can be caused by viruses, bacteria or by fungi. It is by now established that patients that recover from an infectious disease are known to carry therapeutic factors in their blood. These may take the form of immunoglobulins, antibodies or other factors. If a blood donation is taken from a recovering patient, then the plasma component of this donation may be enriched with a range of therapeutic factors that can help to control the pathogen. In convalescent plasma therapy, this plasma is donated to a patient suffering from the disease such that the therapeutic agents can be introduced into the blood stream of the patient where they help to control the symptoms and limit the progression of the disease. This process has been shown to have considerable therapeutic benefit in both viral and bacterial diseases such as Ebola, pertussis, swine flu, bird flu, HIV, SARS, Hepatitis, South American haemorrhagic fevers, influenza and diphtheria amongst others.

In modern medical systems, this separation process is carried out in centralised processing facilities where the blood is spun in a centrifuge which fractionates the blood into various layers which are then separated in subsequent processing steps. The various components are then redistributed to the clinics and hospitals for end use. Such a system requires complex logistical support, refrigerated transport systems and elaborate processing equipment. Such facilities are not always available in developing or emerging nations, in disaster relief situations or in military combat situations. Therefore, there is a requirement for a simple method to separate blood into plasma and cellular components without any of the complex support systems mentioned above.

A conventional device for separating or fractionating blood into single and/or groups of its components in the form of a sterilizable system can comprise a filter arrangement which, by means of filter elements dispersed therein, forms an inlet chamber and an outlet chamber, and a receptacle for cellular blood components and a receptacle for blood plasma, where the inlet chamber of the filter arrangement is connected by conduit on the entry side to a blood source and is connected by conduit on the exit side to a receptacle for cellular components of blood, and where the outlet chamber is connected by conduit on the exit side to a receptacle for blood plasma.

A conventional device of this type is disclosed in European Patent No 1089778 B1. The device is used, solely by utilizing gravity in a filter arrangement, for fractionating blood into its cellular components with erythrocytes on the one hand and plasma with immunoglobulins on the other hand, and for collecting in separate receptacles. Devices of this type are used for processing blood in the event of autologous blood donation but also for the processing of blood during blood donation campaigns. A steam sterilisable device of this kind is disclosed in US Patent No. 8540879.

A further conventional device is described in WO2014/016198 A1, which is distinguished by the fact that the system is divided into at least a first and second sterile packed individual subsystems. These subsystems are connected via Luer-Lock connectors to assemble the subsystems into a working device. It should be noted that such a system may not represent a sterile component when assembled. Furthermore, these conventional devices fail to provide a number of key features that may be used in the preparation of blood plasma from whole blood in the most efficient manner. The extraction of plasma in these devices is based on a slow, drip feed from a single region. The red blood cell receptacle is also known to contain significant quantities of plasma after the filtration and this liquid is not fully removed. Accordingly, it is an object of the present invention to provide a blood fractioning device which overcomes these limitations and is further optimised for convalescent plasma therapy.

### SUMMARY OF THE INVENTION

The object is achieved by a blood fractioning device having the features of claim 1, claim 8 or claim 9.

The invention provides according to a first aspect a gravity based blood fractioning device comprising a component for receiving whole blood which is connected to the inlet chamber of a filter and wherein said filter possesses separate outlet chambers connecting to a receptacle for cellular blood components and a receptacle for blood plasma and wherein each receptacle is connected to said filter via a conduit and wherein the conduit connected to the cellular blood component receptacle is of sufficient length to allow said receptacle to be raised up sufficiently high as to reverse the flow of cellular blood components back through the filter a second time to obtain a second degree of filtration of the cellular blood components and thereby increase the volume of plasma extracted.

In a possible embodiment the conduit connected to the cellular blood component receptacle has a length in a range between 30 cm and 80 cm, preferably between 40 cm and 60 cm and most preferably between 45 cm and 55 cm.

The invention provides according to a second aspect a device for fractionating blood comprising a filter arrangement which, forms an inlet chamber and an outlet chamber, and a receptacle for cellular blood components and a receptacle for blood plasma, the inlet chamber of the filter arrangement having (i) an inlet chamber entry side connected by a first conduit to a blood source and (ii) an inlet chamber exit side connected via a second conduit to the receptacle for cellular components of the blood, wherein blood enters the inlet chamber, passes through the filter element to separate the blood plasma from the blood, and exits into the second conduit, the blood plasma so separated being collected in the outlet chamber for separate removal, the outlet chamber having an outlet chamber exit side connected by a third conduit to the receptacle for blood plasma, wherein the fibres that compose the filter element are so disposed as to form a U-shape and wherein the lowest point on said U-shape is located sufficiently close to the outlet chamber exit side to produce a continuously flowing fluid through the meniscus rather than a drip feed flow of plasma.

The invention provides according to a third aspect a gravity based device for fractionating blood consisting of a component for receiving whole blood which is connected to the inlet chamber of a filter and wherein said filter possesses separate outlet chambers connecting to a receptacle for cellular blood components and a receptacle for blood plasma and wherein each receptacle is connected to said filter via a conduit and wherein the component for receiving whole blood consists of a closed blood spike such that the entire device consists of a sealed internal volume. The blood fractioning device is comprised in a possible embodiment of a filter arrangement which forms an inlet chamber and an outlet chamber, and a receptacle for cellular blood components and a receptacle for blood plasma where the inlet chamber of the filter arrangement having (i) an inlet chamber entry side connected by a first conduit to a blood source and (ii) an inlet chamber exit side connected via a second conduit to the receptacle for cellular components of the blood, wherein blood enters the inlet chamber, passes through the filter element to separate the blood plasma from the blood, and exits into the second conduit, the blood plasma so separated being collected in the outlet chamber for separate removal, the outlet chamber having an outlet chamber exit side connected by a third conduit to the receptacle for blood plasma.

If required, a leukocyte separating filter or a leukocyte depletion filter can be disposed in the conduit between the blood source and the filter arrangement. It has proved to be particularly advantageous for the leukocyte depletion filter to be disposed in the conduit on the entry side of the inlet chamber of the filter arrangement between the filter arrangement and the blood source and/or the hydrophobic or hydrophilic filter associated with this conduit, as is likewise provided by the invention.

The filter elements of the filter arrangement are expediently configured as hollow fibre filters with micropores and consist in particular of polyethersulfone or sulfonated polyethersulfone, so that the invention provides for the filter elements to be hollow fibre filters, in particular made of material having micropores, preferably polyethersulfone. The pore size of the fibres can be chosen so as to prevent the passage of cellular components through the pores while allowing immunoglobulins, antibodies and other factors involved in convalescent plasma therapy to pass through the pores.

In a preferred arrangement, the fibres are so disposed as to form a U-shape, where the lowest part of the U-shape lies adjacent to the outlet chamber exit side. Alternatively, the fibres may be disposed to create more than one turn and form a W, circular or corkscrew pattern.

In previous systems, as the blood passed through the system, the plasma component would pass through the pores of the device and collect on the outer surface of the fibres. As the volume of plasma built up on the fibre surface, a drop would form and this would eventually increase in volume until it became large enough to fall from the device onto the base of the outlet chamber, where it would be collected and allowed to flow into the plasma receptacle. It has been found that if the fibres are placed in close proximity to the base of the outlet chamber, then a large drop does not have to form. Instead, the liquid on the fibre surface can contact the surface of the outlet chamber thus producing a continuous flow of liquid from the fibre surface to the outlet chamber and into the plasma receptacle. This provides a steady, smoother and quicker transfer of plasma. To produce this effect, the fibres have to be sufficiently close to the outlet chamber wall surface to prevent large drops from coalescing on the fibre surface and to ensure that the surface tension of the liquid is sufficient to cause liquid plasma to bridge the gap between the fibre and the surface. In practice, this is best achieved by placing the fibre either in contact with the surface or within 2 mm of the base of the outlet chamber. This ensures that the meniscus formed will connect the fibre to the surface and allow for efficient flow of plasma from the fibre to the plasma receptacle.

To allow for efficient flow, the fibres are first wetted with a suitable liquid. In a preferred embodiment, the liquid is 0.9% saline solution. Adding 30 - 50 ml of saline to the filter is sufficient to achieve wetting. Surprisingly, it has been found that exposing the saline loaded filter to microwave radiation increases the wetting of the fibres and provides for a more homogeneous and repeatable wetting of the fibres and therefore enhances the operation of the filter. To achieve this, the saline is added, the device sealed and the filter is then exposed to microwave radiation for a brief time. The entire device can then be sterilised using steam, gamma radiation or electron beam sterilisation techniques.

In a preferred embodiment, the blood source is whole blood from a donor and is supplied in a standard anti-coagulated blood bag with one or more access ports. The connection is made to the whole blood donor bag using a blood spike present on the end of the conduit that is connected to the filter inlet of the device. The blood spike may be supplied in a vented or a sealed cap. Alternatively, the device may connect to the blood source using Luer-locks. For convenience, after the blood spike is inserted into the blood bag the flow of fluid is controlled using a pinch clamp located adjacent to the blood spike or Luer-Lock.

Under normal operation the donor blood bags is hung from a fastening point, such as a wall hook, nail or transfusion stand. With the spike inserted, when ready, the filtration process can be started by removing the pinch clamp. As the blood source is raised above the filter which hangs below, the blood begins to pass through the filter and is separated into plasma and cellular components. When this first filtration is near completion there is still measurable plasma present in the receptacle containing the cellular components. To optimise the extraction of further plasma from the cellular component mix, the flow into the filter can now be revered. To achieve this, the blood source donor bag is lowered to the same height as the filter or to just below it. This stops the flow of blood into the cellular component receptacle. Simultaneously the cellular component receptacle is raised to a sufficient height to such that gravity causes the cell rich media to flow back into the filter where a second filtration can occur and additional plasma can be captured. As the blood exits the filter it is then collected in the original blood donor source bag. Alternatively, a fresh blood bag can be used to collect the cellular components after the second filtration by swapping the original blood source bag for a fresh blood bag.

In order to effectively raise the receptacle containing the cell rich components, the length of the conduit connecting the receptacle to the filter has to be extended relative to that found in traditional devices. In a preferred embodiment, the conduit is the same length as that of the conduit connecting the blood source to the other side of the filter. These components may be between 30 and 80 cm, or preferably between 40 and 60 cm and most preferably between 45 and 55 cm.

In a further embodiment, the device is supplied with a holder strap or frame which supports the filter from the same fastening point as the donor bag and which is dimensioned to be of sufficient length to allow the filter to hand at the optimum height below the blood source and thereby provide a fixed gravitational head to the unit. This ensures that the weight from the filter is not carried by the blood spike unit and thereby relieves strain on that tubing. In addition, the holder may also contain additional straps or frame elements that are designed to support the receptacles for the plasma and cellular components. These additional supports ensure that the plasma receptacle is placed below the filter to encourage efficient drainage of the plasma away from the fibres and into the receptacle. The support strap or frame can also hold the cellular component receptacle at an appropriate height during the initial filtration and can also provide a second support point if the cellular component receptacle is to be raised up in order to generate a second filtration by reversing the fluid flow back through the same filter.

When used for convalescent therapy, or any other application wherein the plasma is the preferred component produced by the separation process, then the cellular components can be transfused back into the original donor. This ensures that the donor has an adequate supply of red blood cells and allows for a more frequent donation of plasma.

In such a situation, the receptacle for receiving the cellular materials is preferably a blood bag that is devoid of anticoagulant. While many blood bags contain anticoagulant, the bags chosen for this application should not contain any such materials. This ensures that the cellular component does not contain high levels of anticoagulant and can therefore be transfused back into the donor with no risk of complications. Furthermore, if the cellular component is stored in a bag without anticoagulant, then it has been found to be stable and suitable for transfusion for at least six hours after collection. It should be noted that the original source of blood is likely to be a blood bag that does contain anticoagulant. As the blood travels through the filter, some of the anticoagulant will end up in the cellular component receptacle and some may end up in the plasma receptacle. However, the levels present in these receptacles remain suitable for transfusion provided no additional anticoagulant is present in either receptacle.

As the blood fractioning device is designed for use in situations that include disaster relief and combat conditions, the area in which it is used may not be a sterile operating room. As such, the outer part of the blood fractioning device may be contaminated by microbes after the sterile packaging is opened. To prevent contamination of the blood components that are transferred through the internal volume, the blood fractioning device may be deployed as a sealed or closed loop system. To achieve this, the blood spike is enclosed in a sealed or non-vented cap and all other components are shipped in a pre-assembled format where all junctions are preferably welded or fused in a suitable manner to create a seal that is impervious to microbes. This ensures that when blood flows through the system, it cannot be contaminated by microbes present on the outer surface of the device. As the inner surface is sterilised and sealed, there are no internal sources of pathogenic microbes. It should also be noted that the plasma component is cell free and will therefore be free of cell borne pathogens that might be carried in the blood cells. Having such a sealed system ensures that both the plasma receptacle and the cellular component receptacle are maintained free of pathogens internally and this allows the blood components to be transfused into patients without further treatment.

## Claims

1. A gravity based blood fractioning device comprising
a component for receiving whole blood which is connected to an inlet chamber of a filter and wherein said filter possesses separate outlet chambers connecting to a receptacle for cellular blood components and a receptacle for blood plasma and
wherein each receptacle is connected to said filter via a conduit and wherein the conduit connected to the cellular blood component receptacle is of sufficient length to allow said receptacle to be raised up sufficiently high as to reverse the flow of cellular blood components back through the filter a second time to obtain a second degree of filtration of the cellular blood components and thereby increase the volume of plasma extracted.

2. The gravity based blood fractioning device according to claim 1 wherein the conduit connected to the cellular blood component receptacle comprises a range between 30 cm and 80 cm preferably between 40 cm and 60 cm and most preferably between 45 cm and 55 cm.

3. The device according to claim 1 or 2 wherein the cellular material exiting from the filter after the second filtration is collected in a further receptacle connected to the component for receiving whole blood.

4. The device according to claim 1 or 2 wherein the cellular material exiting from the filter after the second filtration is collected in a different receptacle from that used to deliver whole blood during the first filtration.

5. The device according to claim 1 or 2 wherein the receptacles for receiving plasma or for receiving the cellular component do not initially contain anticoagulant.

6. The device according toclaim 1 or 2 wherein the component for receiving blood is a closed spike, an open spike or Luer lock.

7. The device according to one of the preceding claims 1 to 6, wherein the conduit connecting the component for receiving blood to said filter inlet is an equivalent length to the conduit connecting said receptacle for cellular blood components.

8. The device according to one of the preceding claims 1 to 7, wherein the device is supplied with a system to hold each component at an appropriate height during each stage of the process.

9. A device for fractionating blood comprising
a filter arrangement which, forms an inlet chamber and an outlet chamber, and
a receptacle for cellular blood components and a receptacle for blood plasma,
the inlet chamber of the filter arrangement having (i) an inlet chamber entry side connected by a first conduit to a blood source and (ii) an inlet chamber exit side connected via a second conduit to the receptacle for cellular components of the blood,
wherein blood enters the inlet chamber, passes through the filter element to separate the blood plasma from the blood, and exits into the second conduit, the blood plasma so separated being collected in the outlet chamber for separate removal, the outlet chamber having an outlet chamber exit side connected by a third conduit to the receptacle for blood plasma,
wherein the fibres that compose the filter element are so disposed as to form a U-shape and wherein the lowest point on said U-shape is located sufficiently close to the outlet chamber exit side to produce a continuously flowing fluid through the meniscus rather than a drip feed flow of plasma.

10. A gravity based device for fractionating blood consisting of
a component for receiving whole blood which is connected to the inlet chamber of a filter and wherein said filter possesses separate outlet chambers connecting to a receptacle for cellular blood components and a receptacle for blood plasma and
wherein each receptacle is connected to said filter via a conduit and wherein the component for receiving whole blood consists of a closed blood spike such that the entire device consists of a sealed internal volume.

11. The device according to claim 10 such that the internal volume of the device can remain sterile even if the outer surface is contaminated with microbes.

12. The device according to one of the preceding claims 1 to 11 which can be sterilised using one of gamma sterilisation, steam sterilisation or electron beam sterilisation.

13. The device according to one of the preceding claims 1 to 12, wherein the fibres that comprise the filter are first wetted with saline and then exposed to microwave radiation to enhance the wetting of the fibres by the saline.

14. The device according to one of the preceding claims 1 to 13 which also includes pinch clamps to control the flow of fluid.

15. The device according to one of the preceding claims 1 to 14 which can be used to prepare plasma for convalescent plasma therapy.

16. The device according to one of the claims 1 to 15 which can produce convalescent plasma therapy for the treatment of viral infections, including Ebola, swine flu, bird flu, HIV, SARS, Hepatitis, South American haemorrhagic fevers, influenza and diphtheria.

17. The device according to one of the preceding claims 1 to 16 which can produce convalescent plasma therapy for the treatment of bacterial or fungal infections.

18. The device according to one of the preceding claims 1 to 17, wherein the filter is chosen to have pores of sufficient size to allow immunoglobulins, antibodies and other therapeutic factors to enter the plasma receptacle while preventing larger components such as cellular material from entering the plasma receptacle and hence preventing cellular-borne pathogens from entering the plasma receptacle.

19. The device according to one of the claims 1 to 18 which can separate whole blood from a donor into a plasma component for donation to a second individual and a cellular component that can be returned to the original donor within six hours.
